(19) Europäisches Patentamt
European Patent Office
Office européen des brevets

(11) Numéro de publication: **0 210 107**
**A1**

(12) # DEMANDE DE BREVET EUROPEEN

(21) Numéro de dépôt: 86401608.4

(22) Date de dépôt: 18.07.86

(51) Int. Cl.⁴: G 01 N 33/542
G 01 N 33/557, G 01 N 33/18

(30) Priorité: 26.07.85 FR 8511446

(43) Date de publication de la demande:
28.01.87 Bulletin 87/5

(84) Etats contractants désignés:
AT BE CH DE GB IT LI LU NL SE

(71) Demandeur: LYONNAISE DES EAUX Société Anonyme
52 rue de Lisbonne
F-75008 Paris(FR)

(71) Demandeur: Société Anonyme dite: IMMUNOTECH
70, route Léon-Lachamp Case 915 Luminy
F-13288 - Marseille Cedex 09(FR)

(72) Inventeur: DeLaage, Michel
16 rue A. Thiers
F-13001 Marseille(FR)

(72) Inventeur: Prince, Paul
Bâtiment Roumanille Le Félibrige
F-13009 Marseille(FR)

(72) Inventeur: Mallevialle, Joel
Lot 97 Domaine du Bois de Saint Chéron
F-27740 Breuilpont(FR)

(72) Inventeur: Cognet, Louis
9 bis route de Croissy
F-78110 Le Vesinet(FR)

(74) Mandataire: Roger-Petit, Georges et al,
Office Blétry 2, boulevard de Strasbourg
F-75010 Paris(FR)

(54) **Procédé de dosage immunologique en continu de composés organiques dans un fluide en circulation, et dispositif de mise en oeuvre.**

(57) Procédé de dosage immunologique en continu de composés organiques à caractère antigénique dans un fluide en circulation (1), dans lequel on fait passer dans un circuit (3) fermé par un filtre d'échange (2) une partie aliquote du fluide à analyser, le circuit comportant au moins une cartouche (4) d'immunoabsorbant fixant un anticorps approprié en équilibre avec une concentration connue d'un traceur de même caractère antigénique que le composé à doser et on détecte les variations de la concentration en traceur à l'aide d'un détecteur (5).

FIG_1

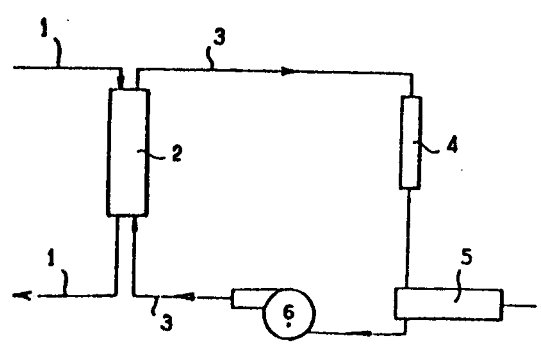


EP 0 210 107 A1

L'invention concerne un procédé de dosage immunologique en continu de produits organiques (médicaments résiduels, pesticides, par exemple) dans un fluide en circulation (eau par exemple).

Du fait de la présence croissante de produits organiques tels que pesticides dans les eaux naturelles, les pouvoirs publics tant sur le plan national qu'international ont été amenés à imposer ou recommander des seuils limites de tolérance de ces produits dans les eaux potables. Bien que l'élimination des pesticides soit réalisée de façon satisfaisante par l'emploi de filtres à charbon actif, éventuellement associé à un traitement par l'ozone, le problème du dosage de ces produits dans l'eau reste mal résolu car il exige des équipements lourds et du personnel expérimenté en raison des concentrations infinitésimales qu'il faut mesurer (de l'ordre du µg/l). En outre la gamme de produits à rechercher s'élargit de jour en jour et chaque produit demande la mise au point d'une méthode de dosage particulière.

On a donc cherché à mettre au point un procédé de dosage automatique pouvant reconnaître un produit ou des familles étendues de pesticides, permettant tant la surveillance des eaux potables après traitement que le contrôle des procédés industriels de traitement des eaux naturelles.

Pour ce faire, on a cherché à utiliser les larges possibilités qu'offre l'analyse immunologique bien connue dans le domaine du diagnostic clinique. On sait que la réaction anticorps-antigène est extrêmement sensible et permet de détecter la présence de substances aux concentrations des hormones dans le sang. Or ces concentrations sont comparables à celles des pesticides et produits toxiques dans l'eau (de l'ordre du µg/l et moins à quelques mg/l généralement. Les méthodes classiques de dosage de ces produits

(chromatographie en phase gazeuse, chromatographie liquide à haute performance, dosage de l'activité anticholinestérasique) demandent une extraction afin de concentrer le produit à rechercher, ne peuvent pas être utilisées en continu de façon simple et leur sensibilité n'est pas toujours bonne.

Un certain nombre d'essais ont été faits afin de doser les agents polluants par voie immunologique mais n'ont pas abouti à des procédés exploitables sur le plan industriel. En outre ces essais ont cherché à calquer les modes d'analyse sur ceux de l'immunoanalyse des hormones ou des médicaments dans le sang pour laquelle on recherche un temps de réponse réduit avec des échantillons à analyser de faible volume.

L'invention fournit donc un procédé de dosage immunologique en continu de composés organiques à caractère antigénique dans un fluide en circulation, dans lequel une partie aliquote du fluide est introduite dans un circuit fermé comportant une cartouche contenant un anticorps approprié en équilibre avec une quantité connue d'un traceur macromoléculaire de même caractère antigénique que le ou les composés à doser et on détecte la variation de la répartition du traceur (proportion traceur lié/traceur libre).

On peut utiliser principalement trois types de traceurs permettant une détection simple : les traceurs radioactifs, les traceurs fluorescents et les traceurs enzymatiques.

L'anticorps utilisé pourra être spécifique d'un composé organique donné ou d'une famille de composés. De même on peut envisager l'utilisation d'anticorps polyclonaux ou monoclonaux.

L'utilisation de plusieurs cartouches dans le circuit, contenant chacune un anticorps particulier permet le dosage séparé de plusieurs composés organiques donnés.

Le dosage global de plusieurs composés organiques pourra être effectué si la cartouche contient plusieurs anticorps.

Il est également fourni un dispositif de mise en oeuvre du procédé comportant un filtre d'échange sur le fluide en circulation, un circuit fermé par un filtre d'échange formant une boucle autour du filtre d'échange et comportant une cartouche contenant un immunoadsorbant sur lequel est fixé l'anticorps, un

détecteur, et une pompe de mise en circulation de fluide dans le circuit fermé, le traceur antigénique étant conçu de manière à être captif dans le circuit fermé soit à l'état fixé sur l'anticorps soit à l'état libre dans le fluide.

L'invention va maintenant être décrite en détail en se référant aux dessins annexés dans lesquels :

La figure 1 est un schéma de principe du dispositif de mise en oeuvre du procédé selon l'invention ;

La figure 2 est un schéma d'un autre dispositif de mise en oeuvre ; et

La figure 3 est un graphique montrant la relation entre la valeur théorique et la valeur analytique de la quantité de composé en fonction du temps.

La figure 1 illustre le schéma de principe du procédé de dosage selon l'invention.

Le fluide en circulation dans la conduite 1 (de l'eau par exemple) pénètre dans un filtre d'échange 2 (membrane de dialyse, filtre) permettant le passage de petites molécules et retenant celles de poids moléculaire élevé. Sur ce filtre d'échange 2, est monté un circuit fermé 3 comportant une cartouche 4 dans laquelle l'anticorps est fixé sur un immunoadsorbant, un détecteur 5 et une pompe 6 pour compenser la perte de charge dans l'immunoadsorbant.

Le circuit 3 contient une quantité connue de traceur à caractère antigénique. A l'équilibre, une partie du traceur est fixé sur l'anticorps lié à l'immunoadsorbant en raison de la réaction anticorps-antigène et l'autre partie du traceur est libre et circule dans le circuit 3, à l'état dissous dans l'eau. Ce traceur constitué d'une molécule de haut poids moléculaire est retenu par le filtre 2 et est donc captif dans le circuit 3. Le détecteur donne alors une valeur de référence.

Lorsque l'eau à analyser contient un produit organique à détecter (pesticide par exemple) constituant un antigène, celui-ci pénètre dans le circuit 3 à travers le filtre 2 et entre en compétition avec le traveur fixé sur l'immunoadsorbant pour la réaction antigène - anticorps. La quantité de traceur en circulation est modifiée et le détecteur détecte cette modification.

La figure 2 représente un autre dispositif de mise en oeuvre. De l'eau circule dans la canalisation 11. Une partie de l'eau traverse un premier filtre 12 comportant un filtre retenant les molécules de poids moléculaire élevé (supérieur à 50 000 par exemple). L'eau ainsi filtrée passe dans la canalisation 13 puis est injectée dans le circuit 15. L'eau contenant l'antigène (dont le poids moléculaire lui permet de traverser les filtres 12 et 14) circule alors dans le circuit 15 comportant plusieurs cartouches 16 d'immunoadsorbant (deux étant représentées sur la figure 2, 16a et 16b). Des détecteurs 17 sont reliés aux cartouches 16 et la figure 2 montre deux détecteurs 17a et 17b correspondant respectivement aux cartouches 16a et 16b. Le filtre 14 ayant une valeur de coupure de 10 000 assure l'élimination de l'excédent d'eau du circuit 15 en retenant le traceur ( masse moléculaire > 10 000).Des pompes P assurent la circulation de l'eau pour compenser les pertes de charge dues aux filtrations. L'eau rejetée par les filtres 12 et 14 est envoyée à l'égoût.

On va maintenant étudier plus en détail les composants du dispositif ainsi que les caractéristiques cinétiques des réactions antigène - anticorps dans la cartouche d'immunoadsorbant.

Le principe du dosage immunologique est une réaction antigène - anticorps, pour laquelle il faut avoir en présence un antigène et un anticorps spécifique.

Toute molécule de poids moléculaire égal ou supérieur à 200 peut constituer un antigène et peut être dosée par un processus immunologique à condition de pouvoir synthétiser l'anticorps complémentaire. Dans le cas d'un antigène de masse moléculaire inférieure à 10 000, la production d'anticorps nécessite la synthèse du couple antigène-macromolécule,

Des études antérieures ont montré que les pesticides appartenant aux familles des organochlorés, des organophosphorés, des carbamates, des phénoxyacétiques, des triazines, des benzimidazoles et thiophanates, et des dinitrophénols et des biphénylpolychlorés, pour ne reprendre que les principales familles de pesticides selon les normes de la Communauté Economique Européenne, ont donné lieu à la production d'anticorps après fixation sur une substance de poids moléculaire élevé (polymère,

protéine...). La fixation de l'antigène sur l'haptène (substance polymère ou protidique) pour l'obtention de l'anticorps n'est pas toujours simple à réaliser mais un grand nombre de conjugaisons sont résolues ou en voie de résolution à l'heure actuelle. Il est possible de recourir aux anticorps monoclonaux pour améliorer les caractéristiques (affinité, reproductibilité, etc.).

La seule limite au procédé est donc la fabrication d'un anticorps correspondant au produit organique que l'on désire doser, quelle que soit son activité biologique (médicament, pesticide , produit toxique autre, etc...).

L'anticorps est ensuite fixé sur un immunoadsorbant.Les imunoadsorbants sont des supports organiques ou minéraux (poudre ou billes de verre, ...) permettant le greffage d'anticorps par réaction covalente ou d'adsorption. Il existe un certain nombre de supports immunoadsorbants mais l'un des plus utilisés est le produit "Affigel" (commercialisé par BIORAD) qui est un polysaccharide activé à l'N-hydroxysuccinimide et sur lequel on peut greffer des anticorps par réaction covalente. Les immunoadsorbants ont une capacité de fixation de 1 à 50 mg d'anticorps pour 10 ml d'immunoadsorbant. En pratique on utilisera de l'ordre de 2 nanomoles d'anticorps dans le circuit. L'immunoadsorbant est contenu dans une cartouche , généralement une petite colonne dont le diamètre permet un débit correspondant à celui existant dans le circuit.

L'antigène après couplage avec une substance polymère ou protidique (par exemple, albumine) doit ensuite être transformé en traceur. Ceci est obtenu en couplant l'antigène avec le groupement traceur approprié (substituant radioactif, fluorescent ou enzymatique) à la méthode de détection utilisée. Les traceurs les plus utilisés sont les molécules marquées à l'iode radioactif ($^{125}I$) et l'on sait à l'heure actuelle synthétiser les traceurs iodés même avec des molécules fragiles, par exemple par couplage de ces dernières avec des molécules déjà iodées.

Le détecteur est bien entendu dépendant du type de traceur utilisé.

Dans le cas d'un traceur radioactif (par exemple sérum-abumine marquée à $^{125}I$ et portant le motif antigénique), on enregistre le rayonnement radioactif à l'aide d'une sonde

appropriée (par exemple ensemble Nardeux IMP10 + SMX) soit au niveau de l'immunoadsorbant et l'on observe une diminution de la radioactivité (due à la diminution de la quantité de traceur lié) soit sur le circuit (par exemple en y introduisant une cartouche vide) et l'on observe alors une augmentation de la radioactivité (augmentation de la quantité de traceur en circulation). L'utilisation d'une cartouche vide pour la détermination de la variation permet de la mesurer plus précisément : il est plus facile de mesurer une augmentation, même faible, à partir d'une valeur faible, (par exemple une augmentation de 2 % à partir d'une valeur de 5 %) que de mesurer la même diminution absolue (2 %) à partir d'une valeur élevée (par exemple 95%).

Dans le cas d'un traceur enzymatique où l'antigène traceur est couplé à une enzyme, la détection s'effectue à l'extérieur de l'immunoadsorbant par injection de substrat et détermination de l'activité enzymatique, par exemple détection colorimétrique différentielle pour une détection en continu du traceur libre en circulation dans le circuit 15.

Le traceur peut également comporter un élément donnant lieu à une fluorescence et le détecteur est alors un détecteur de fluorescence.

Bien entendu l'invention n'est pas limitée à l'utilisation d'un type particulier de détection et d'autres techniques de détection peuvent être envisagées. On peut citer par exemple les détecteurs à électrodes enzymatiques qui permettent de détecter la présence des pesticides.

L'un des paramètres importants du dispositif de dosage est le temps de réponse, c'est-à-dire le temps nécessaire pour obtenir une valeur correspondant à la valeur réelle de la teneur en composés organiques.

Du fait que le composé à doser entre en compétition avec le traceur pour la fixation sur les sites anticorps liés à l'immunoadsorbant, il faut un certain temps pour que le détecteur donne une valeur. Le temps de réponse est fonction de la cinétique de la réaction antigène-anticorps que l'on décrira brièvement ci-dessous.

Cette réaction s'écrit

$$\text{Anticorps + antigène} \underset{kd}{\overset{ka}{\rightleftharpoons}} \text{complexe anticorps-antigène}$$

où $k_a$ est une constante de vitesse d'association de second ordre et $k_d$ une constante de vitesse de dissociation de premier ordre. Ces études décrites dans la littérature montrent que les constantes de vitesse d'association sont toujours élevées alors que les constantes de vitesse de dissociation peuvent varier dans d'assez larges limites.

Le graphique de la figure 3 représente en traits pleins la valeur théorique de la concentration en antigène dans le fluide à analyser (unités arbitraires en ordonnées) en fonction du temps et la courbe en traits interrompus donne la valeur mesurée par le détecteur. On constate que la valeur détectée croît rapidement et jusqu'à une valeur asymptotique. Connaissant les constantes de vitesse d'association et de dissociation d'un complexe antigène-anticorps donné, on peut calculer la vitesse de la variation de la concentration en traceur et en déduire la concentration de la substance à doser. Il sera en général possible de choisir le couple anticorps- antigène traceur pour que le temps de réponse soit de l'ordre d'une heure.

L'existence de ce temps de réponse n'est pas particulièrement gênante dans les principales applications du procédé, qui sont la surveillance de la concentration en pesticides des eaux naturelles et des eaux potables après traitement industriel. En effet le détecteur peut être relié à une alarme qui est déclenchée lorsque la valeur mesurée atteint une valeur prédéterminée, même si la valeur mesurée n'a pas atteint son niveau maximum.

Bien entendu, le procédé de dosage et le dispositif approprié s'appliquent au dosage de très nombreux composés organiques, qu'ils s'agissent de médicaments, produits toxiques, pesticides, résidus industriels, dans la mesure où ils peuvent constituer un antigène et où l'on peut synthétiser un anticorps et un traceur antigénique correspondant . De même d'autres fluides que l'eau peuvent ainsi être analysés.

En outre, l'utilisation de plusieurs cartouches contenant chacune un seul anticorps permet le dosage séparé mais simultané de plusieurs composés organiques, alors que l'utilisation d'une seule cartouche contenant plusieurs anticorps permet le dosage global de plusieurs composés organiques.

Le circuit fermé peut être modifié par des vannes provoquant son fonctionnement périodique et non plus continu. Dans ce cas, on déterminera la quantité de composé organique recueillie dans un temps donné, avec un effet de concentration qui permet de doser des quantités encore plus faibles de composé organique.

On va décrire un exemple de dosage utilisant le procédé et le dispositif de l'invention, le produit à doser étant le médicament appelé Intensain.

Exemple L'Intensain ou carbochromène a la formule

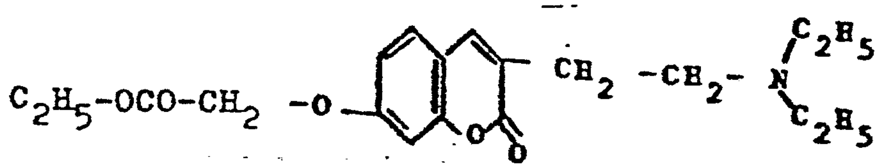

qui existe également sous forme acide.

1°) Obtention de l'anticorps anti-Intensain.

L'Intensain sous forme acide carboxylique est couplé à de la sérum albumine bovine (BSA) par réaction au chloroformiate d'éthyle selon le mode opératoire suivant :

On dissout 4 mg d'Intensain dans 500 µl de diméthylformamide (DMF) anhydre et on ajoute 20 µl de diéthylamine dilués au 1/11$^{e}$ dans le DMF puis 20 µl de chloroformiate d'éthyle dilué au 1/16$^{e}$. On laisse sous agitation pendant 10 minutes puis on ajoute 200 µl de cette solution (solution A) à 1,5 ml d'une solution de BSA à 10 mg/ml et on agite vigoureusement. Après filtration l'analyse spectrale du produit obtenu montre qu'il comporte 5,3 moles d'Intensain par mole de BSA.

L'injection de ce composé à des lapins en présence d'adjuvant de Freund provoque la production d'anticorps anti-Intensain.

2°) Fixation de l'anticorps sur l'immunoadsorbant

Un antisérum de lapin obtenu par injection d'anticorps anti-Intensain est couplé sur Affigel 10 selon le mode opératoire suivant. On lave 10 ml d'Affigel 10 successivement avec 30 ml

d'isopropanol, 200 ml d'$H_2O$ et 200 ml de $NaHCO_3$ 0,1 M à pH 8,5 puis on les met en contact à 4°C avec 0,6 ml d'antisérum dilué dans 5 ml de $NaHCO_3$ 0,1 M à pH 8,5. On laisse 18 heures sous agitation à 4°C et on bloque par réaction avec 2 ml de glycinamide 0,5 M à pH 7,2 pendant 1 heure à 4°C.

Le rendement de couplage est de 55 % et le produit obtenu contient environ 2 nanomoles d'anticorps spécifique pour 5 ml de gel.

3°) Obtention du traceur.

On couple de l'Intensain à de la BSA selon le mode opératoire utilisé pour la préparation d'anticorps anti-Intensain mais en utilisant une proportion différente d'Intensain.

100 µl de la solution A et 1,6 ml de BSA à 10 mg/ml.

On obtient un complexe comportant 3,3 moles d'Intensain par mole de BSA.

Sur le complexe obtenu par couplage (3,3 moles d'Intensain/mole de BSA), on effectue le marquage à l'iode radioactif selon un mode opératoire classique (chloramine T) et on obtient un complexe radioactif Intensain -BSA-I.

4°) Réalisation du circuit de dosage.

On monte un circuit selon le schéma de la figure 1 sauf que l'on place le détecteur de radioactivité sur la cartouche d'immunoadsorbant. On introduit dans le circuit la quantité désirée de traceur et on laisse le circuit s'équilibrer pendant environ 18 heures.

Puis lorsqu'on injecte de l'Intensain dans la conduite 1 en quantités croissantes, on note une décroissance de la radioactivité dans la cartouche d'immunoadsorbant.

## REVENDICATIONS

1. Procédé de dosage immunologique en continu de composés organiques à caractère antigénique dans un fluide en circulation, caractérisé en ce qu'une partie aliquote du fluide contenant le ou les composés à doser est introduite dans un circuit fermé comportant une cartouche contenant un anticorps approprié en équilibre avec une quantité connue d'un traceur macromoléculaire de même caractère antigénique que le composé à doser et que l'on détecte les variations de la répartition du traceur (traceur lié/traceur libre).

2. Procédé selon la revendication 1, caractérisé en ce que le traceur est un traceur radioactif et que la détection se fait à l'aide d'un compteur de radioactivité.

3. Procédé selon la revendication 1, caractérisé en ce que le traceur est un traceur enzymatique et que la détection se fait par voie enzymatique.

4. Procédé selon la revendication 1, caractérisé en ce que le traceur est un traceur fluorescent et que la détection se fait par fluorescence.

5. Procédé selon l'une quelconque des revendications 1 à 4, caractérisé en ce que l'anticorps est spécifique d'un seul composé organique.

6. Procédé selon l'une quelconque des revendications 1 à 4, caractérisé en ce que l'anticorps est spécifique d'une famille de composés organiques.

7. Procédé selon l'une quelconque des revendications 1 à 4, caractérisé en ce qu'on place dans ce circuit plusieurs cartouches d'immunoadsorbant comportant des anticorps différents et que l'on détecte pour chaque cartouche la variation de concentration en traceur correspondant.

8. Procédé selon l'une quelconque des revendications 1 à 4, caractérisé en ce qu'on dose globalement plusieurs composés organiques en faisant passer le fluide dans une cartouche contenant plusieurs anticorps.

9. Dispositif de mise en oeuvre du procédé selon l'une quelconque des revendications 1 à 4, caractérisé en ce qu'il comporte une conduite (1) de fluide en circulation, un filtre d'échange (2) entre la conduite (1) et un circuit fermé (3) comportant au moins une cartouche d'immunoadsorbant (4) contenant un anticorps, un détecteur (5) et une pompe de circulation (6), le traceur antigénique étant captif dans le circuit fermé (3).

10. Dispositif selon la revendication 9, caractérisé en ce qu'il comporte plusieurs cartouches d'immunoadsorbant (16a, 16b) à chacune desquelles correspondent un traceur de nature différente et un nombre correspondant de détecteurs (17a, 17b).

FIG_1

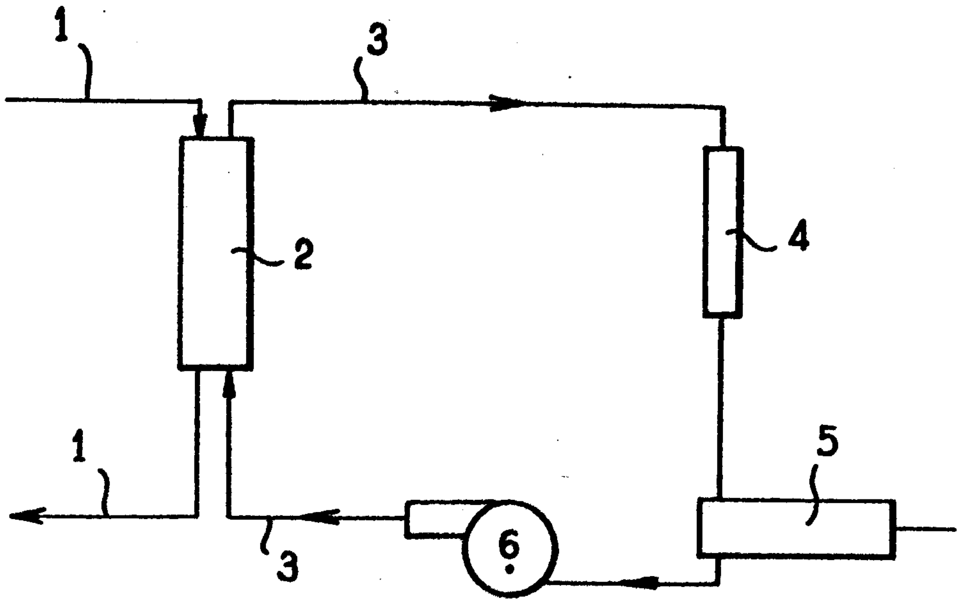
1
3
2
4
1
5
6
3

FIG_3

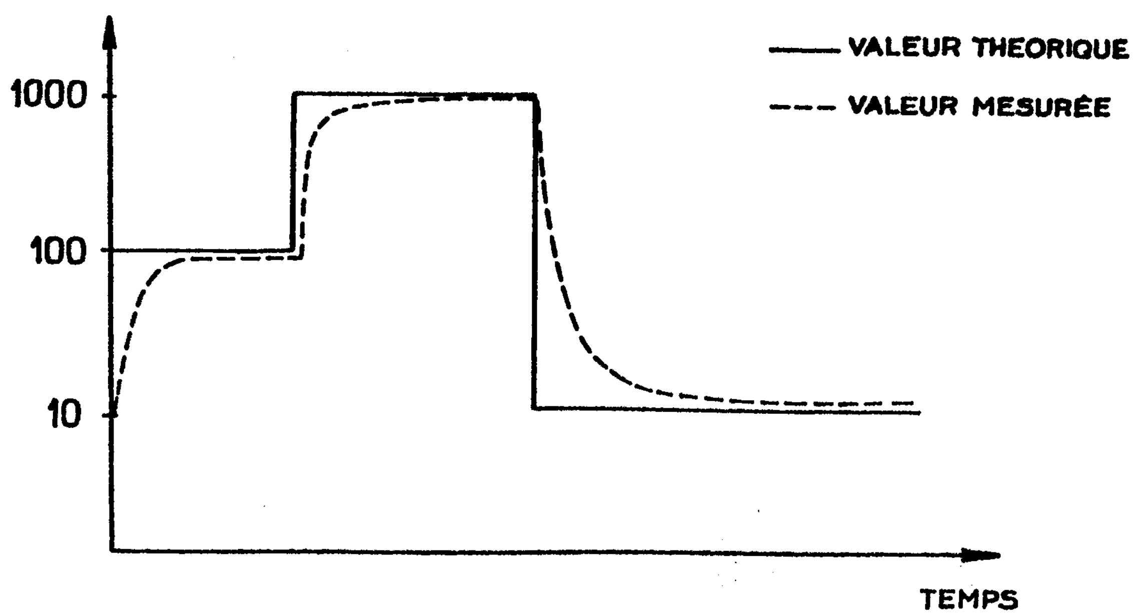
1000
100
10
VALEUR THEORIQUE
VALEUR MESURÉE
TEMPS

FIG. 2

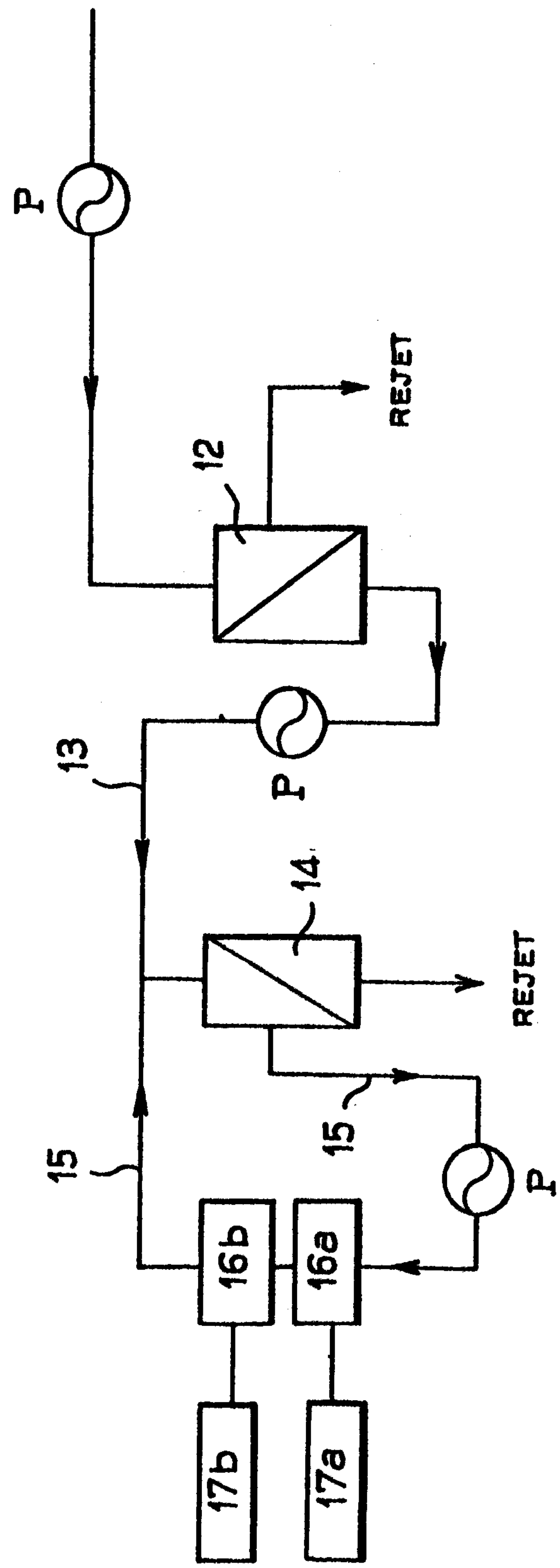

Office européen des brevets

**0210107**

Numero de la demande

# RAPPORT DE RECHERCHE EUROPEENNE

EP 86 40 1608

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | FR-A-2 365 124 (BOEHRINGER MANNHEIM GmbH) * Page 3, ligne 18 - page 5, ligne 11; page 6, ligne 37 - page 11, ligne 27; revendications 1-4,8,9,13 * | 1-5,9 | G 01 N 33/543<br>G 01 N 33/557<br>G 01 N 33/18 |
| A | FR-A-2 430 013 (SOCIETE ANONYME SEBIA, J.P. PINON et al.) * En entier * | 1,3,5,8,9 | |
| A | FR-A-2 272 102 (TECHNICON INSTRUMENTS CORP.) * Page 2, ligne 19 - page 3, ligne 16; page 5, ligne 37 - page 7, ligne 1; exemple 5; revendications * | 1-4,9 | |
| | | | DOMAINES TECHNIQUES RECHERCHES (Int Cl 4) |
| A | FR-A-2 357 900 (BECTON DICKINSON AND CO.) * Page 1, ligne 1 - page 3, ligne 20; page 5, ligne 10 - page 6, ligne 26; revendications 1,4,5,7,13 * | 1-4,9 | G 01 N |
| A | EP-A-0 094 770 (BECTON DICKINSON AND CO.) * Résumé; page 4, ligne 14 - page 7, ligne 23; revendications 1,9,10; figures 1-6 * | 1,2,8 | |

Le présent rapport de recherche a été établi pour toutes les revendications

| Lieu de la recherche | Date d'achèvement de la recherche | Examinateur |
|---|---|---|
| LA HAYE | 16-09-1986 | HITCHEN C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l invention
E : document de brevet antérieur, mais publié à la date de dépôt ou après cette date
D : cité dans la demande
L : cité pour d'autres raisons

& : membre de la même famille, document correspondant

OEB Form 1503 03 82